Europäisches Patentamt
European Patent Office
Office européen des brevets

(19) (11) Veröffentlichungsnummer: **0 092 721**
**A1**

(12) # EUROPÄISCHE PATENTANMELDUNG

(21) Anmeldenummer: 83103472.3

(22) Anmeldetag: 11.04.83

(51) Int. Cl.³: C 07 D 307/86
A 01 N 47/24
//C07C143/74, C07C145/00

(30) Priorität: 23.04.82 DE 3215256

(43) Veröffentlichungstag der Anmeldung: 02.11.83 Patentblatt 83/44

(84) Benannte Vertragsstaaten: AT BE CH DE FR GB IT LI NL SE

(71) Anmelder: BAYER AG
Zentralbereich Patente, Marken und Lizenzen
D-5090 Leverkusen 1, Bayerwerk(DE)

(72) Erfinder: Hartmann, Alfons, Dr.
Bruchrandweg 42
D-5024 Pulheim 3(DE)

(72) Erfinder: Heywang, Gerhard, Dr.
Nittumer Weg 4
D-5060 Bergisch Gladbach 2(DE)

(72) Erfinder: Kühle, Engelbert, Dr.
V.-Bodelschwingh-Strasse 42
D-5060 Bergisch Gladbach 2(DE)

(72) Erfinder: Hammann, Ingeborg, Dr.
Lutherstrasse 22
D-4330 Mülheim/Ruhr(DE)

(72) Erfinder: Homeyer, Bernhard, Dr.
Obere Strasse 28
D-5090 Leverkusen 3(DE)

(54) **N-sulfenylierte Carbamate, Verfahren zu ihrer Herstellung sowie ihre Verwendung als Schädlingsbekämpfungsmittel.**

(57) Die vorliegende Erfindung betrifft neue N-sulfenylierte Carbamate der allgemeinen Formel (I)

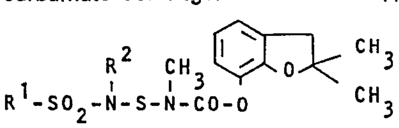

(I)

in welcher
- $R^1$ für gegenbenenfalls durch Halogen, Nitril oder Nitro substituiertes, geradkettiges oder verzweigtes Alkyl mit 1-8 C-Atomen oder für gegebenenfalls durch Halogen, Nitril oder Nitro substituiertes Cycloalkyl mit 3-7 C-Atomen und
- $R^2$ für geradkettiges oder verzweigtes Alkyl mit 1-12 C-Atomen oder für Cycloalkyl mit 3-7 C-Atomen

steht.

Sie werden erhalten, indem man Carbamidsäurefluoride der Formel (II)

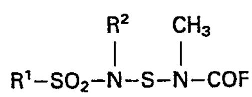

(II)

mit 2,3-Dihydro-2,2-dimethyl-7-benzofuranol der Formel (III)

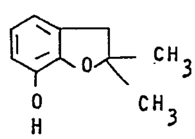

(III)

umsetzt.

Die erfindungsgemäßen Verbindungen eignen sich zur Schädlingsbekämpfung.

EP 0 092 721 A1

Croydon Printing Company Ltd.

0092721

BAYER AKTIENGESELLSCHAFT 5090 Leverkusen, Bayerwerk

Zentralbereich
Patente, Marken und Lizenzen Rt/bc/c
Ia

# N-sulfenylierte Carbamate, Verfahren zu ihrer Herstellung sowie ihre Verwendung als Schädlingsbekämpfungsmittel

Die vorliegende Erfindung betrifft neue N-sulfenylierte Carbamate, ein Verfahren zu ihrer Herstellung und ihre Verwendung als Schädlingsbekämpfungsmittel.

Es ist bereits bekannt geworden, daß N-sulfenylierte Carbamate insektizide, akarizide und nematizide Eigenschaften haben (vgl. DOS 2 344 175, 2 434 184, 2 600 981 und 2 609 830). Die Wirkung der dort beschriebenen Verbindungen ist jedoch, vor allem bei niedrigen Aufwandmengen, nicht immer voll befriedigend.

Es wurden die neuen N-sulfenylierten Carbamate der allgemeinen Formel (I) gefunden

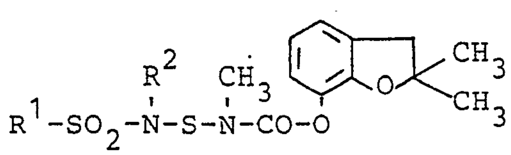

(I)

in welcher

$R^1$ für gegebenenfalls durch Halogen, Nitril oder Nitro substituiertes, geradkettiges oder verzweigtes Alkyl mit 1-8 C-Atomen oder für gegebenenfalls durch Halogen, Nitril oder Nitro substituiertes Cycloalkyl mit 3-7 C-Atomen und

$R^2$ für geradkettiges oder verzweigtes Alkyl mit 1-12 C-Atomen oder für Cycloalkyl mit 3-7 C-Atomen

steht.

Weiterhin wurde gefunden, daß man die neuen N-sulfenylierten Carbamate der Formel (I)

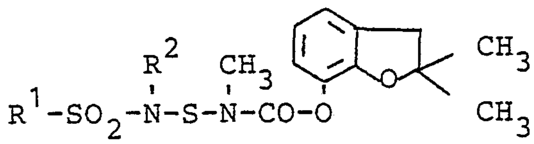

(I)

in welcher

$R^1$ für gegebenenfalls durch Halogen, Nitril oder Nitro substituiertes, geradkettiges oder verzweigtes Alkyl mit 1-8 C-Atomen oder für gegebenenfalls durch Halogen, Nitril oder Nitro substituiertes Cycloalkyl mit 3-7 C-Atomen und

$R^2$ für geradkettiges oder verzweigtes Alkyl mit 1-12 C-Atomen oder für Cycloalkyl mit 3-7 C-Atomen

steht,

erhält, wenn man Carbamidsäurefluoride der Formel (II)

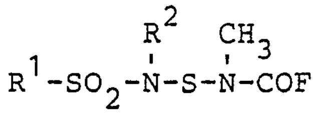

(II)

in welcher
$R^1$ und $R^2$ die oben angegebene Bedeutung haben,

mit 2,3-Dihydro-2,2-dimethyl-7-benzofuranol der Formel (III)

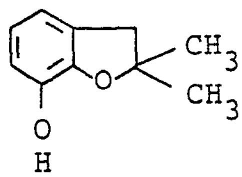

(III)

gegebenenfalls in Gegenwart eines säurebindenden Mittels und/oder eines Verdünnungsmittels umsetzt.

Die erfindungsgemäßen Verbindungen eignen sich zur Schädlingsbekämpfung.

Es ist ausgesprochen überraschend, daß die erfindungsgemäßen Verbindungen eine höhere insektizide, akarizide und nematizide Wirkung zeigen als die aus dem Stand der Technik bekannten N-sulfenylierten Carbamate. Die erfindungsgemäßen Stoffe stellen somit eine Bereicherung der Technik dar.

Bevorzugt sind Verbindungen der Formel (I), in welcher

$R^1$ für $C_{1-4}$-Alkyl steht, das gegebenenfalls durch Halogen substituiert ist und
$R^2$ für $C_{1-4}$-Alkyl steht.

Besonders bevorzugt sind Verbindungen der Formel (I), in welcher

$R^1$ für Methyl steht, das gegebenenfalls durch Halogen, insbesondere Chlor, substituiert ist und

$R^2$ für Methyl und Butyl steht.

Verwendet man N-Methyl-N-(methansulfonsäure-n-butyl-amid-N'-sulfenyl)-carbamidsäurefluorid und 2,3-Dihydro-2,2-dimethyl-7-benzofuranol als Ausgangsstoffe, so läßt sich der Reaktionsablauf durch das folgende Formelschema wiedergeben:

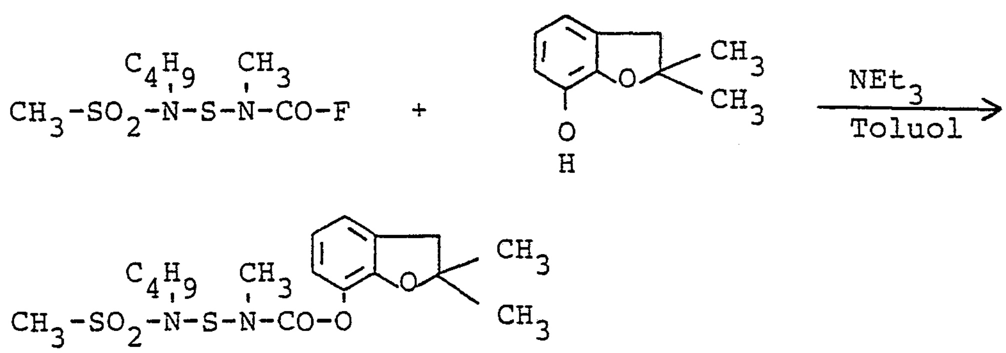


Die eingesetzten Carbamidsäurefluoride der Formel (II) sind neu. Sie können hergestellt werden, indem zunächst Sulfonsäurechloride mit primären Aminen zu den entsprechenden Sulfonsäureamiden umgesetzt werden. Diese werden dann mit Dischwefeldichlorid in Disulfide übergeführt. Die Spaltung dieser Disulfide mit Chlor liefert die entsprechenden Sulfenchloride, die mit N-Methylcarbamidsäurefluoriden zu den Carbamidsäurefluoriden der Formel (II) umgesetzt werden können. Das Verfahren ist an sich aus DE-OS 2 254 359 bekannt und wird wie dort beschrieben durchgeführt.
Vorzugsweise verwendet werden Carbamidsäurefluoride der Formel (II), bei der $R^1$ für Methyl, Ethyl, Propyl, iso-Propyl, Butyl, iso-Butyl, tert.-Butyl, Pentyl, Chlormethyl, 2,2,2-Trifluorethyl, 2-Nitrilethyl, 2-Nitroethyl, 3-Chlorpropyl und $R^2$ für Methyl, Ethyl, Propyl, iso-Propyl, -Butyl, iso-Butyl, tert.-Butyl, Pentyl, Dodecyl, Cyclohexyl, steht.

Als Verdünnungsmittel eignen sich alle inerten organischen Lösungsmittel. Hierzu gehören Ether wie Diethylether, Dioxan oder Tetrahydrofuran, Kohlenwasserstoffe wie Dichlormethan, Chloroform oder Chlorbenzol, weiterhin Nitrile und Ester sowie Gemische aus diesen Lösungsmitteln.

Zur Bindung des bei der Reaktion entstehenden Fluorwasserstoffs setzt man dem Reaktionsgemisch vorzugsweise eine tertiäre organische Base wie z.B. Triethylamin oder Dimethylbenzylamin zu.

Die Reaktionstemperaturen können in einem größeren Bereich variiert werden. Im allgemeinen arbeitet man zwischen 0 und 100°C, vorzugsweise bei 20 bis 60°C.

Gewöhnlich werden die Reaktionspartner in äquimolaren Mengenverhältnissen eingesetzt, doch auch die Verwendung einer Komponente im Überschuß ist möglich.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Tineola bisseliella, Tinea pellionella
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

## Herstellungsbeispiele

### I. Herstellung der Ausgangsprodukte

Ia) Methansulfonsäurebutylamid; $CH_3SO_2-NH-C_4H_9$

Zu einer Lösung von 229 g (2 mol) Methansulfonsäurechlorid in 1 l Toluol tropft man bei 0°C 292 g (4 mol) Butylamin. Man rührt 4 Stunden nach. Nach dem Zufügen von 1 l Ether wird der Niederschlag abfiltriert und die organische Phase eingeengt. Der Rückstand wiegt 298 g (98 % der Theorie).
Analog erhält man Chlormethansulfonsäure-N-methylamid; $ClCH_2SO_2NHCH_3$; $Kp_{0,1}$ 117°C; Ausbeute 46 %.

Ib) Bis-(N-methansulfonyl-N-butyl-amino)-disulfid

$$\begin{array}{c} \quad\quad\quad C_4H_9 \quad\quad\; C_4H_9 \\ CH_3-SO_2-N-S-S-N-SO_2-CH_3 \end{array}$$

302 g (2 mol) des wie oben beschrieben erhaltenen Öls und 202 g (2 mol) Triethylamin werden in 1 l Toluol gelöst. Bei Raumtemperatur tropft man 135 g (1 mol) Dischwefeldichlorid zu. Man rührt 6 Stunden bei Raumtemperatur und 1 Stunde bei 40°C nach, schüttelt zweimal mit Wasser und einmal mit wäßriger Ammoniumchloridlösung aus, trocknet die organische Phase über Magnesiumsulfat und engt im Vakuum ein. Es bleiben 286 g (78 % der Theorie) als Öl zurück.

Analog erhält man Bis-(N-methyl-N-chlormethansulfonylamino)-disulfid; Fp. 81°C; Ausbeute 85 %.

Ic) N-Methylsulfonyl-N-butyl-amino-sulfenylchlorid

$$\begin{array}{c} C_4H_9 \\ CH_3SO_2-N-S-Cl \end{array}$$

In eine Lösung von 286 g (0,79 mol) des wie voran beschrieben hergestellten Disulfids in 1 l Tetrachlorkohlenstoff werden bei Raumtemperatur 66 g (0,79 mol) Chlorgas eingeleitet. Anschließend rührt man 4 Stunden nach und vertreibt überschüssiges Chlor, indem man durch das Reaktionsgemisch einen Stickstoffstrom leitet und engt dann das Reaktionsgemisch ein. Man erhält 294 g Sulfenchlorid in Form eines orangefarbenen Öls (86 % der Theorie).
Analog erhält man N-Chlormethylsulfonyl-N-methyl-amino-sulfenylchlorid; Ausbeute 100 %.

Id) N-Methyl-N-(methansulfonsäurebutylamid-N'-sulfenyl)-carbamidsäurefluorid

$$\begin{array}{c} C_4H_9\;CH_3 \\ CH_3-SO_2-N-S-N-COF \end{array}$$

Zu einer Lösung von 178 g (0,82 mol) des wie voran beschriebenen gewonnenen Sulfenchlorids und 65 g (0,82 mol) N-Methyl-carbamidsäurefluorid in 1 l

Toluol tropft man bei Raumtemperatur 113 ml (0,82 mol) Triethylamin. Man rührt 6 Stunden bei 25°C und 1 Stunde bei 40°C nach, schüttelt zweimal mit Wasser und zweimal mit 10 %iger wäßriger Ammoniumchloridlösung aus, trocknet die organische Phase über Magnesiumsulfat und engt schließlich im Wasserstrahlvakuum ein. Man erhält 158 g Carbamidsäurefluorid in Form eines braunen Öls.
$n_D^{20}$ = 1,4852 (74 % der Theorie).
Analog erhält man N-(Methansulfonsäure-methylamid-N'-sulfenyl)-N-methylcarbamidsäurefluorid;
$n_D^{20}$ = 1,4992; Ausbeute 94 %.

II. Herstellung der neuen Verbindungen der Formel (I)

1) 2,3-Dihydro-2,2-dimethyl-7-benzofuranyl-N-(methansulfonsäure-butylamid-N'-sulfenyl)-N-methyl-carbamat

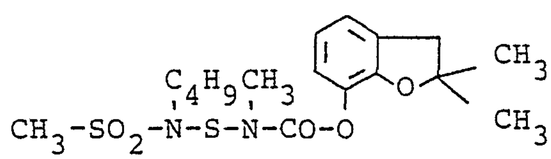

Zu 158 g (0,61 mol) N-Methyl-N-(methansulfonsäure-butylamid-N'-sulfenyl)-carbamidsäurefluorid und 100 g (0,61 mol) 2,3-Dihydro-2,2-dimethyl-7-benzofuranol in 1 l Toluol tropft man bei 25°C (0,61 mol) Triethylamin. Man rührt eine Stunde bei dieser Temperatur und 2 Stunden bei 50 bis 60°C. Das Reaktionsgemisch wird mit Wasser geschüttelt. Die organische Phase wird abgetrennt und zweimal mit 10 %iger wäßriger Ammoniumchloridlösung ausgewaschen, anschließend über Magnesiumsulfat getrocknet und im Vakuum des Lösungsmittels abdestilliert. Es bleiben 202 g öliger Rückstand (86 % der Theorie). $n_D^{20}$ : 1,5281.

Analog erhält man ausgehend von Chlormethansulfonsäuremethylamid:

2) 2,3-Dihydro-2,2-dimethyl-7-benzofuranyl-N-(chlormethansulfonsäure-methylamid-N'-sulfenyl)-N-methylcarbamat

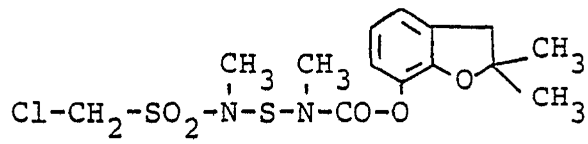

Beispiel A

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheiden ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1

Beispiel B

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1,2

0092721

Beispiel

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1,2

Beispiel

Doralis-Test (systemische Wirkung)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1

0092721

Beispiel

Laphygma-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1,2

Patentansprüche

1) N-sulfenylierte Carbamate der allgemeinen Formel (I)

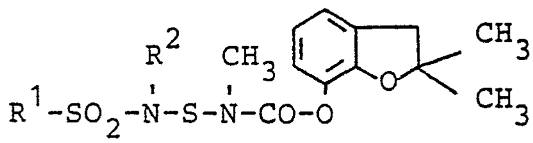

(I)

in welcher

$R^1$ für gegebenenfalls durch Halogen, Nitril oder Nitro substituiertes, geradkettiges oder verzweigtes Alkyl mit 1-8 C-Atomen oder für gegebenenfalls durch Halogen, Nitril oder Nitro substituiertes Cycloalkyl mit 3-7 C-Atomen und

$R^2$ für geradkettiges oder verzweigtes Alkyl mit 1-12 C-Atomen oder für Cycloalkyl mit 3-7 C-Atomen steht.

2) Verfahren zur Herstellung von N-sulfenylierten Carbamaten der Formel (I)

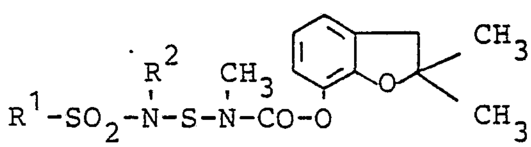

(I)

in welcher

$R^1$ für gegebenenfalls durch Halogen, Nitril oder Nitro substituiertes, geradkettiges oder verzweigtes Alkyl mit 1-8 C-Atomen oder für gegebenenfalls durch Halogen, Nitril oder Nitro substituiertes Cycloalkyl mit 3-7 C-Atomen und

$R^2$ für geradkettiges oder verzweigtes Alkyl mit 1-12 C-Atomen oder für Cycloalkyl mit 3-7 C-Atomen steht,

dadurch gekennzeichnet, daß man Carbamidsäurefluoride der Formel (II)

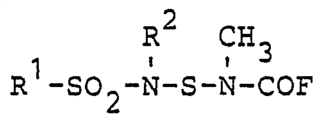 (II)

in welcher
$R^1$ und $R^2$ die oben angegebene Bedeutung haben,

mit 2,3-Dihydro-2,2-dimethyl-7-benzofuranol der Formel (III)

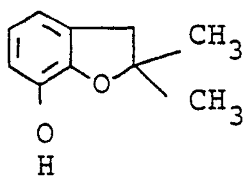 (III)

gegebenenfalls in Gegenwart eines säurebindenden Mittels und/oder eines Verdünnungsmittels umsetzt.

3) Verbindungen der Formel (I) gemäß Anspruch 1, dadurch gekennzeichnet, daß

$R^1$ für $C_{1-4}$-Alkyl steht, das gegebenenfalls durch Halogen substituiert ist und
$R^2$ für $C_{1-4}$-Alkyl steht.

4) Verbindungen der Formel (I) gemäß Anspruch 1, dadurch gekennzeichnet, daß

$R^1$ für Methyl steht, das gegebenenfalls durch Halogen, insbesondere Chlor, substituiert ist und $R^2$ für Methyl und Butyl steht.

5) Schädlingsbekämpfungsmittel, gekennzeichnet durch einen Gehalt an mindestens einem N-sulfenylierten Carbamat der Formel (I).

6) Verwendung von N-sulfenylierten Carbamaten der Formel (I) zur Bekämpfung von Schädlingen.

7) Verfahren zur Bekämpfung von Schädlingen, dadurch gekennzeichnet, daß man N-sulfenylierte Carbamate der Formel (I) auf Schädlinge und/oder ihren Lebensraum einwirken läßt.

8) Verfahren zur Herstellung von Schädlingsbekämpfungsmitteln, dadurch gekennzeichnet, daß man N-sulfenylierte Carbamate der Formel (I) mit Streckmitteln und/oder oberflächenaktiven Mitteln vermischt.

0092721

Europäisches Patentamt

# EUROPÄISCHER RECHERCHENBERICHT

Nummer der Anmeldung

EP 83 10 3472

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | DE-A-2 344 175 (BAYER AG.) * Insgesamt * | 1-8 | C 07 D 307/86<br>A 01 N 47/24 //<br>C 07 C 143/74<br>C 07 C 145/00 |
| | ----- | | |
| | | | **RECHERCHIERTE SACHGEBIETE (Int. Cl. 3)** |
| | | | C 07 D 307/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 08-07-1983 | ALLARD M.S. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82